# EUROPEAN PATENT APPLICATION

(11) **EP 4 303 210 A1**
(43) Date of publication of application: **10.01.2024**
(21) Application number: 22763357.5
(22) Date of filing: 02.03.2022
(51) Int. Cl.: C07C 381/00, C07D 213/71

(54) **METHOD FOR PRODUCING PENTAFLUOROSULFANYL GROUP-CONTAINING ARYL COMPOUND**

(30) Priority: 02.03.2021 JP 2021032818
(71) Applicant: AGC INC., Chiyoda-ku, Tokyo 1008405 (JP); The University of Tokyo, Bunkyo-ku, Tokyo 113-8654 (JP)
(72) Inventor: OKAZOE Takashi, Tokyo 100-8405 (JP); GATZENMEIER Tim, Tokyo 113-8654 (JP); NOZAKI Kyoko, Tokyo 113-8654 (JP)
(74) Representative: TBK
(86) International application number: PCT/JP2022/008977
(87) International publication number: WO 2022/186304

(57) **Abstract**

The present invention provides a production method capable of efficiently synthesizing an SF₅ group-containing compound, or the like. The present invention is a method for synthesizing an SF₅ group-containing compound represented by the general formula (1) from a thioaryl compound represented by the general formula (2) [A¹ is an aryl group or a heteroaryl group; G¹ is -SH, -SCN, SF₃, -S-S-R¹, -S-CO-R², -SR³, -SF₂-R⁴, -S-Si-(R⁵)₃, -S-PO-(R⁶)₂, (N-phthalimidyl)thio group, or a thianthrenium group (R¹ is an aryl group or a heteroaryl group; R² and R⁵ are an aryl group, a heteroaryl group, an alkyl group, or an alkenyl group; R³ and R⁴ are an alkyl group or an alkenyl group, R⁶ is an aryloxy group, a heteroaryloxy group, an alkyloxy group, or an alkenyloxy group)].

[Chemical Formula 1]

A¹-G¹ (2)

A¹-SF₅ (1)

## Description

### [Technical Field]

The present invention relates to a method for producing a pentafluorosulfanyl group-containing aryl compound in which a pentafluorosulfanyl group is introduced into an aryl group.

Priority is claimed on Japanese Patent Application No. 2021-032818, filed March 2, 2021, the content of which is incorporated herein by reference.

### [Background Art]

The pentafluorosulfanyl (SF₅) group has a relatively small size, high electron-withdrawing property, excellent hydrolytic stability, and improved lipophilicity. Therefore, it is regarded as a "super trifluoromethyl (CF₃) group" with excellent properties. On the other hand, it is difficult to introduce the SF₅ group into existing compounds, and for this reason, despite its high attractiveness, the use of the SF₅ group in the active ingredients of pharmaceuticals and agricultural chemicals, organic materials, etc. has not progressed.

Conventional methods for synthesizing a compound in which the SF₅ group is introduced into an aryl group such as a phenyl group have various drawbacks. For example, there is a method of directly fluorinating an aryl disulfide using fluorine gas (F₂) (Patent Document 1). However, in this method, the benzene ring in the aryl disulfide is also fluorinated when an electron-withdrawing group such as a nitro group is not introduced to the benzene ring. Further, in the another method of obtaining an arylsulfapentafluoride (Ar-SF₅) is fluorination of an aryl disulfide fluorinated using chlorine gas (Cl₂) and a potassium fluoride to obtain an aryltetrafluorosulfanyl chloride (Ar-SF₄Cl), followed by fluorinating this using a zinc fluoride (ZnF₂) or the like (Patent Document 2). In the method, although the benzene ring is not fluorinated even if there is no nitro group or the like, there is a risk of chlorination of the benzene ring. Using silver fluoride (II) (AgF₂), an aryl disulfide can be fluorinated in a Freon refrigerant to obtain an arylsulfatrifluoride (Ar-SF₃), followed by further heating to 130°C to obtain Ar-SFs (Non-Patent Document 1). However, the yield of this method is poor. Another method of synthesizing Ar-SF₅ by fluorinating of an aryl disulfide consists of using a tetraalkylammonium chloride and a xenon (II) fluoride (XeF₂) (Non-Patent Document 2). However, since Ar-SF₄Cl is also co-produced in this method, it is necessary to separate Ar-SF₄Cl.

### [Citation List]

### [Patent Document]

[Patent Document 1] Published Japanese Translation No. 10-507206 of the PCT International Publication
[Patent Document 2] Published Japanese Translation No. 2010-522213 of the PCT International Publication

### [Non-Patent Document]

[Non-Patent Document 1] Sheppard, Communications to the Editor, 1960, vol. 82, p. 4751-4752.
[Non-Patent Document 2] Ou and Janzen, Journal of Fluorine Chemistry, 2000, vol. 101, p. 279-283.

### [Summary of Invention]

### [Technical Problem]

The objective of the present invention is to provide a novel production method capable for efficiently synthesizing pentafluorosulfanyl group-containing aryl compounds in which a pentafluorosulfanyl group is introduced to an aryl group.

### [Solution to Problem]

The present inventors have found that a pentafluorosulfanyl group-containing aryl compound can be synthesized from a thioaryl compound in a single step by using, as a fluorinating agent, a silver (II) fluoride and a tetraalkylammonium halide, and completed the invention.

The present invention is as follows.
[1] A method for producing a pentafluorosulfanyl group-containing aryl compound, comprising:
   synthesizing a pentafluorosulfanyl group-containing aryl compound represented by the following general formula (1) from a thioaryl compound represented by the following general formula (2) by an oxidative fluorination reaction using a divalent or higher valent metal fluoride and an organic salt including a quaternary ammonium cation or a quaternary phosphonium cation,
   [Chemical Formula 1]

   A¹-G¹ (2)

   [in the formula, A¹ is an optionally substituted aryl group or an optionally substituted heteroaryl group; -G¹ is -SH, -SCN, -SF₃, -S-S-R¹ (R¹ is an optionally substituted aryl group or an optionally substituted heteroaryl group), -S-CO-R² (R² is an optionally substituted aryl group, an optionally substituted heteroaryl group, an optionally substituted alkyl group having 1 to 6 carbon atoms or an optionally substituted alkenyl group having 2 to 6 carbon atoms), -S-R³ (R³ is an optionally substituted alkyl group having 1 to 6 carbon atoms or an optionally substituted alkenyl group having 2 to 6 carbon atoms), -SF₂-R⁴ (R⁴ is an optionally substituted alkyl group having 1 to 6 carbon atoms or an optionally substituted alkenyl group having 2 to 6 carbon atoms), -S-Si-(R⁵)₃ (R⁵ is an optionally substituted aryl group, an optionally substituted heteroaryl group, an optionally substituted alkyl group having 1 to 6 carbon atoms, or an optionally substituted alkenyl group having 2 to 6 carbon atoms, and a plurality of R⁵ may be the same or different), -S-PO-(R⁶)₂ (R⁶ is an optionally substituted aryloxy group, an optionally substituted heteroaryloxy group, an optionally substituted alkyloxy group having 1 to 6 carbon atoms, an optionally substituted alkenyloxy group having 2 to 6 carbon atoms, an optionally substituted aryl group, an optionally substituted hetero aryl group, an optionally substituted alkyl group having 1 to 6 carbon atoms, or an optionally substituted alkenyl group having 2 to 6 carbon atoms, and a plurality of R⁶ may be the same or different), an optionally substituted (N-phthalimidyl) thio group, or an optionally substituted thianthrenium group],
      [Chemical Formula 2]

      A¹-SF₅ (1)
   [In the formula, A¹ is the same as described above]
[2] The method for producing a pentafluorosulfanyl group-containing aryl compound according to [1], wherein A¹ is
   an aryl group optionally substituted with one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a cyano group, a fluoroformyl group, an amino group and a nitro group; or
   a heteroaryl group optionally substituted with one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a fluoroformyl group, a cyano group, an amino group and a nitro group.
[3] The method for producing a pentafluorosulfanyl group-containing aryl compound according to [1] or [2], wherein the oxidative fluorination reaction is carried out at -40 to 130°C.
[4] The method for producing a pentafluorosulfanyl group-containing aryl compound according to any one of [1] to [3], wherein a metal produced after the oxidative fluorination reaction is recovered.
[5] The method for producing a pentafluorosulfanyl group-containing aryl compound according to [4], wherein
   the recovered metal is fluorinated to regenerate a divalent or higher valent metal fluoride, and the obtained divalent or higher valent metal fluoride is used again in the oxidative fluorination reaction.
[6] The method for producing a pentafluorosulfanyl group-containing aryl compound according to any one of [1] to [5], wherein the divalent or higher valent metal fluoride is silver (II) fluoride.
[7] The method for producing a pentafluorosulfanyl group-containing aryl compound according to any one of [1] to [6], wherein the organic salt is a tetraalkylammonium halide.

### [Advantageous Effect of Invention]

According to the method of the present invention, the oxidative fluorination of thioaryl compounds can be performed in a single step, and a pentafluorosulfanyl group-containing aryl compound can be efficiently synthesized.

### [Description of Embodiments]

In the present invention and the specification of the present application, "Cₚ₁₋ₚ₂" (p1 and p2 are positive integers satisfying p1 <p2) means a group having p1 to p2 carbon atoms.

In the present invention and the specification of the present application, a "C₁₋₆ alkyl group" is an alkyl group having 1 to 6 carbon atoms, and may be linear or branched. Examples of the C₁₋₆ alkyl group include a methyl group, an ethyl group, a propyl group, an isopropyl group, a butyl group, an isobutyl group, a see-butyl group, a tert-butyl group, a pentyl group, an isopentyl group, a neopentyl group, a tert-pentyl group, a hexyl group and the like.

In the present invention and the specification of the present application, the term "C₁₋₆ alkoxy group" refers to a group in which an oxygen atom is bonded to the terminal end of a C₁₋₆ alkyl group. A C₁₋₆ alkoxy group may be linear or branched. Examples of the C₁₋₆ alkoxy group include a methoxy group, an ethoxy group, a propoxy group, a butoxy group, a tert-butoxy group, a pentyloxy group, a hexyloxy group and the like.

In the present invention and the specification of the present application, the term "C₂₋₆ alkenyl group" refers to a group in which at least one carbon-carbon bond of an alkyl group having 2 to 6 carbon atoms is an unsaturated bond. The C₂₋₆ alkenyl group may be linear or branched. Examples of the C₂₋₆ alkenyl group include a vinyl group, an allyl group, a butenyl group, a pentenyl group, a hexenyl group and the like.

In the present invention and the specification of the present application, the term "C₂₋₇ acyl group" refers to a group in which the hydrocarbon group moiety obtained by removing the carbonyl group from the acyl group is a C₁₋₆ alkyl group, a C₂₋₆ alkenyl group, a 5- to 6-membered aryl group or a 5- to 6-membered heteroaryl group. The hydrocarbon group moiety of the acyl group may be linear or branched. Examples of the C₂₋₇ acyl group include a formyl group, an acetyl group, a propanoyl group, a propenoyl group, a benzoyl group and the like.

In the present invention and the specification of this application, the term "halogen atom" refers to a fluorine atom, a chlorine atom, a bromine atom, or an iodine atom. The term "halogen atom other than a fluorine atom" refers to a chlorine atom, a bromine atom, or an iodine atom. As the "halogen atom other than a fluorine atom", a chlorine atom or a bromine atom is preferable, and a chlorine atom is particularly preferable.

Moreover, hereinafter, the term "compound (n)" refers a compound represented by formula (n).

### <Oxidative fluorination reaction>

In the method for producing a pentafluorosulfanyl group-containing aryl compound (hereinafter may be referred to as "SFs-containing aryl compound") according to the present invention, the sulfur atom bonded to an aryl group in a thioaryl compound is fluorinated by an oxidative fluorination reaction using a divalent or higher valent metal fluoride such as a silver fluoride (II) (AgF₂) and an organic salt such as a tetraalkylammonium halide (hereinafter, may be referred to as "NR¹¹₄X", X represents a halogen atom). If AgF₂ is used alone as a fluorinating agent, the sulfur atoms bonded to the aryl group are fluorinated only up to the SF₃ group (Non-Patent Document 1). In contrast, in the present invention, by using a divalent or higher valent metal fluoride such as AgF₂ in combination with an organic salt such as NR¹¹₄X, the SF₄X group-containing aryl compound obtained during the reaction can be used as it is in the reaction system without isolation and reacted with AgF₂ or the like to fluorinate the SF₄Cl group to the SF₅ group. That is, a desired SF₅-containing aryl compound can be obtained from a thioaryl compound in a single step by using a divalent or higher valent metal fluoride such as AgF₂ and an organic salt such as NR¹¹₄X in combination as a fluorinating agent.

Specifically, in the method for producing an SF₅-containing aryl compound according to the present invention, an SF₅-containing aryl compound represented by the following general formula (1) is synthesized from a thioaryl compound represented by the general formula (2) by an oxidative fluorination reaction using a divalent or higher valent metal fluoride such as AgF₂ and an organic salt such as NR¹¹₄X. The chemical reaction formula when AgF₂ and NR¹¹₄X are used is shown below.

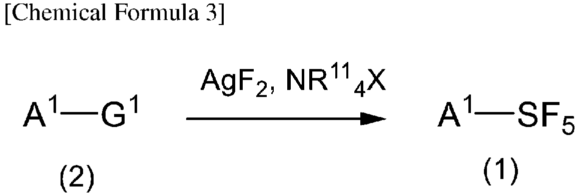

In the general formulas (2) and (1), A¹ is an optionally substituted aryl group or an optionally substituted heteroaryl group. The aryl group is not particularly limited and examples thereof include, a phenyl group, a naphthyl group, an anthryl group, a 9-fluorenyl group and the like, and a phenyl group is particularly preferable. The heteroaryl group is not particularly limited and examples thereof include, a pyridyl group, a pyrazinyl group, a pyrimidinyl group, a pyridazinyl group, a pyrazolyl group, a quinolyl group, an isoquinolyl group, a pyrrolyl group, an imidazolyl group, an indolyl group, a furyl group, a benzofuryl group, a thienyl group, a benzothienyl group, an oxazolyl group, an isoxazolyl group, a thiazolyl group, an isothiazolyl group and the like.

The term "optionally substituted aryl group" refers to a group in which one or a plurality of hydrogen atoms, preferably 1 to 3 hydrogen atoms bonded to the carbon atoms of the aryl group are substituted with other functional groups. Similarly, the term "optionally substituted heteroaryl group" refers to a group in which one or a plurality of hydrogen atoms, preferably 1 to 3 hydrogen atoms bonded to the carbon atoms of the heteroaryl group are substituted with other functional groups. When having two or more substituents, the substituents may be the same or different.

The aryl group and the heteroaryl group for A¹ may have one or two or more substituents in addition to the sulfur atom to be fluorinated. Examples of the substituents include a halogen atom, an alkyl group, a fluorinated alkyl group, an alkenyl group, an alkoxy group, an aryl group, a heteroaryl group, an acyl group, a hydroxy group, a carboxy group, a cyano group, a fluoroformyl group (-C(=O)F), an amino group, a nitro group and the like. The alkyl group is preferably a C₁₋₆ alkyl group, the alkenyl group is preferably a C₂₋₆ alkyl group, the alkoxy group is preferably a C₁₋₆ alkoxy group, and the acyl group is preferably a C₂₋₇ acyl group. The fluorinated alkyl group is preferably a group in which one or two or more hydrogen atoms of a C₁₋₆ alkyl group are substituted with fluorine atoms, and a fully fluorinated C₁₋₆ alkyl group in which all hydrogen atoms are substituted with fluorine atoms is more preferable, and a trifluoromethyl group is particularly preferable. Examples of the aryl group and the heteroaryl group include the aryl groups and the heteroaryl groups for A¹, respectively, and a phenyl group and a pyridyl group are preferable.

The substituent of the aryl group and the heteroaryl group for A¹ may be protected with a protecting group. As the protective group, a group commonly used in organic synthesis can be appropriately used. For example, when the substituent is an amino group, the amino group can have two hydrogen atoms replaced with a tert-butoxycarbonyl group, a benzyloxycarbonyl group, a 9-fluorenylmethyloxycarbonyl group, a 2,2,2-trichloroethoxycarbonyl group, an allyloxycarbonyl group, a trifluoroacetyl group, a phthaloyl group, a p-toluenesulfonyl group or 2-nitrobenzenesulfonyl group before being subjected to an oxidative fluorination reaction. Similarly, when the substituent is a carboxy group, the carboxy group can have a hydrogen atom substituted with a benzyl group or a tert-butyl group.

Since a divalent or higher valent metal fluoride such as AgF₂ and an organic salt such as NR¹¹₄X are used in combination as a fluorinating agent, even when the electron density of the aryl ring is high, the oxidative fluorination reaction of the sulfur atom bonded to the carbon atom of the aryl ring proceeds. Also, even when an electron-withdrawing group such as a nitro group is not bonded to the aryl ring, halogenation of the carbon atom itself of the aryl ring is unlikely to occur. For this reason, in the aryl group of the thioaryl compound (2), even when one or two or more of the carbon atoms other than the carbon atom bonded to the sulfur atom to be fluorinated is substituted with an electron-withdrawing group or substituted with an electron-donating group, fluorination of the aryl ring does not occur, and the desired SF₅-containing aryl compound can be obtained efficiently. The same applies to the heteroaryl ring.

In the general formula (2), G¹ is any one of the following groups. The black circle represents a bonding site.

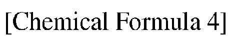

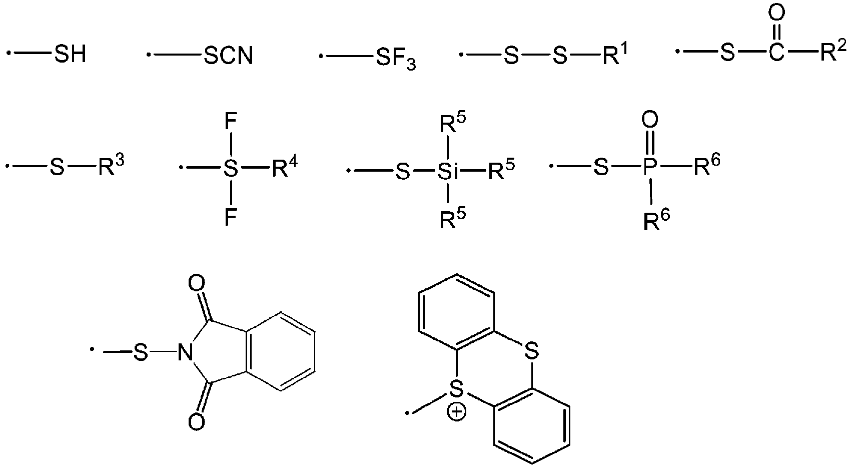

R¹ is an optionally substituted aryl group or an optionally substituted heteroaryl group.

R² is an optionally substituted aryl group, an optionally substituted heteroaryl group, an optionally substituted C₁₋₆ alkyl group or an optionally substituted C₂₋₆ alkenyl group.

R³ is an optionally substituted C₁₋₆ alkyl group or an optionally substituted C₂₋₆ alkenyl group.

R⁴ is an optionally substituted C₁₋₆ alkyl group or an optionally substituted C₂₋₆ alkenyl group.

R⁵ is an optionally substituted aryl group, an optionally substituted heteroaryl group, an optionally substituted C₁₋₆ alkyl group or an optionally substituted C₂₋₆ alkenyl group, and a plurality of R⁵ may be the same or different.

R⁶ is an optionally substituted aryloxy group, an optionally substituted heteroaryloxy group, an optionally substituted C₁₋₆ alkyloxy group, an optionally substituted C₂₋₆ alkenyloxy group, an optionally substituted aryl group, an optionally substituted heteroaryl group, an optionally substituted C₁₋₆ alkyl group or an optionally substituted C₂₋₆ alkenyl group, and a plurality of R⁶ may be the same or different.

The optionally substituted aryl group for R¹, R², R⁵ and R⁶ may be the same as those mentioned for A¹.

The optionally substituted heteroaryl group for R¹, R², R⁵ and R⁶ may be the same as those mentioned for A¹.

In R², R³, R⁴, R⁵, and R⁶, the term "optionally substituted C₁₋₆ alkyl group" refers to a group in which one or a plurality of hydrogen atoms, preferably 1 to 3 hydrogen atoms bonded to the carbon atoms of the C₁₋₆ alkyl group are substituted with other functional groups. Similarly, the term "optionally substituted C₂₋₆ alkenyl group" refers to a group in which one or a plurality of hydrogen atoms, preferably 1 to 3 hydrogen atoms bonded to the carbon atoms of the C₂₋₆ alkenyl group are substituted with other functional groups. When having two or more substituents, the substituents may be the same or different. Examples of the substituents include a halogen atom, an alkyl group, an alkenyl group, an alkoxy group, an aryl group, an acyl group, a hydroxy group, a carboxy group, a cyano group, an amino group, a nitro group and the like. The alkyl group is preferably a C₁₋₆ alkyl group, the alkenyl group is preferably a C₂₋₆ alkyl group, the alkoxy group is preferably a C₁₋₆ alkoxy group, and the acyl group is preferably a C₂₋₇ acyl group.

In R⁶, the optionally substituted aryloxy group may be a group in which the aryl group moiety is the same group as those exemplified as the optionally substituted aryl group for A¹. R⁶ is preferably a phenyloxy group.

In R⁶, the optionally substituted heteroaryloxy group may be a group in which the heteroaryl group moiety is the same group as those exemplified as the optionally substituted heteroaryl group for A¹. R⁶ is preferably a pyridyloxy group.

In R⁶, the optionally substituted C₁₋₆ alkyloxy group may be a group in which the C₁₋₆ alkyl group moiety is the same group as those exemplified as the optionally substituted C₁₋₆ alkyl group for R² and the like. R⁶ is preferably a methyloxy group.

In R⁶, the optionally substituted C₂₋₆ alkenyloxy group may be a group in which the C₂₋₆ alkenyl group moiety is the same group as those exemplified as the optionally substituted C₂₋₆ alkenyl group for R² and the like. R⁶ is preferably a vinyloxy group.

When G¹ is a (N-phthalimidyl)thio group, 1 to 3 hydrogen atoms of the benzene ring in the (N-phthalimidyl)thio group may be substituted with a substituent. The substituent is not particularly limited as long as it is a group that does not inhibit the fluorination reaction, and examples thereof include the same substituents as those exemplified as the "optionally substituted C₁₋₆ alkyl group" for R² and the like. A plurality of the substituents may all be the same or different. G¹ is preferably an unsubstituted (N-phthalimidyl)thio group.

When G¹ is a thianthrenium group, 1 to 4 hydrogen atoms of one or both of the two benzene rings in the thianthrenium group may be substituted with a substituent. The substituent is not particularly limited as long as it is a group that does not inhibit the fluorination reaction, and examples thereof include the same substituents as those exemplified as the "optionally substituted C₁₋₆ alkyl group for R² and the like". A plurality of the substituents may all be the same or different. G¹ is preferably an unsubstituted thianthrenium group or a halogen-substituted thianthrenium group.

The thioaryl compound (2) may be a compound having a plurality of the structures with G¹ bound to A¹ in one molecule. For example, a compound having a structure in which A¹ is a biphenyl group and G¹ is linked to both of the two benzene rings is also included in the thioaryl compound (2). Thus, when one molecule has a plurality of G¹ groups bonded to the aryl ring or the heteroaryl ring, all G¹ groups are converted to pentafluorosulfanyl groups by the oxidative fluorination reaction.

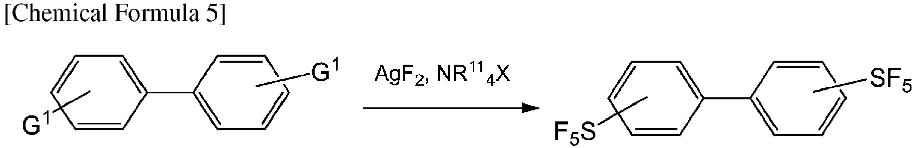

Specific examples of the thioaryl compound (2) include the following compounds. Compounds in which one or a plurality of substituents are introduced into the benzene ring of these compounds are also preferable as the thioaryl compound (2) used in the present invention. Examples of the substituents include a halogen atom, an alkyl group, an alkenyl group, an alkoxy group, an aryl group, an acyl group, a hydroxy group, a carboxy group, a cyano group, an amino group, a nitro group and the like.

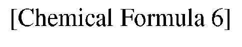

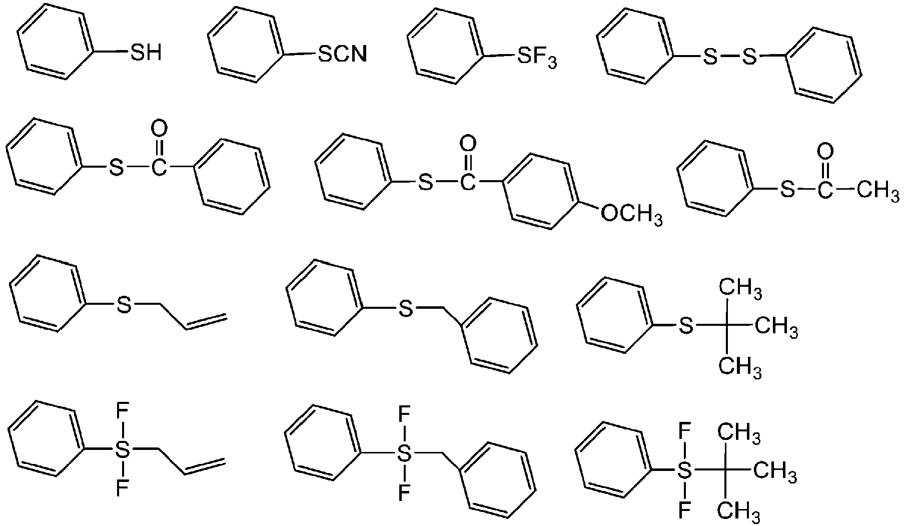

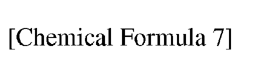

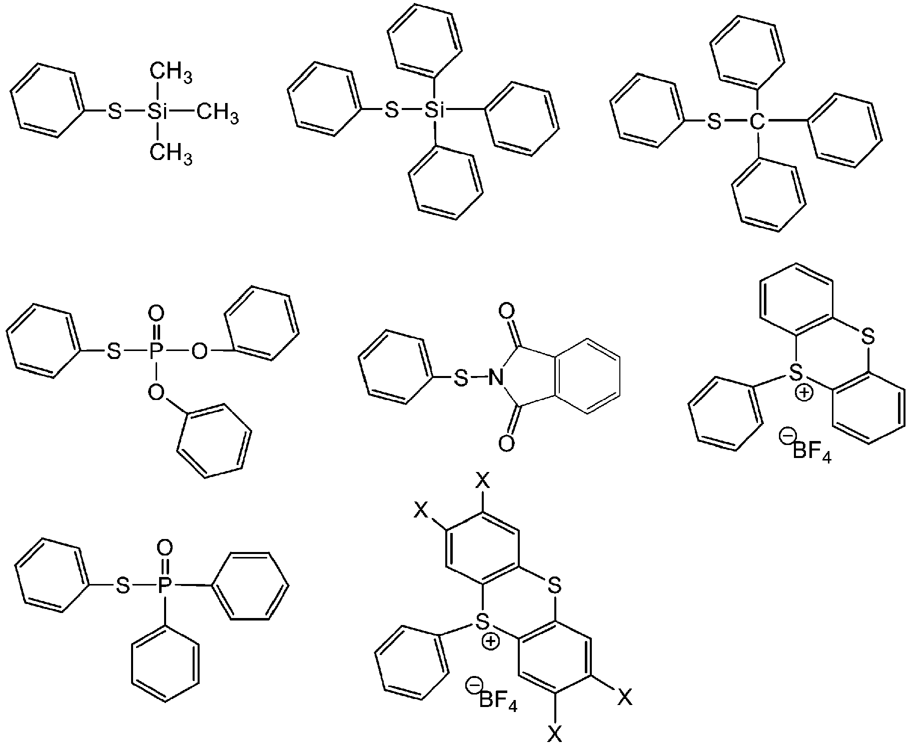

Examples of the divalent or higher valent metal fluoride used as a fluorinating agent in the oxidative fluorination reaction include fluorides of a first transition element, a second transition element, or a third transition element. Specifically, the fluorinating agent used in the present invention is preferably a divalent or higher valent fluoride of silver, niobium, manganese, cobalt, copper, hafnium, tantalum or cerium, such as AgF₂, manganese fluoride (III) (MnF₃), cobalt (III) fluoride (CoF₃), copper (II) fluoride (CuF₂), niobium (V) fluoride (NbF₅), hafnium (V) fluoride (HfF₅), tantalum fluoride (TaF₅), cerium (IV) fluoride (CeF4)) and the like. As the divalent or higher valent metal fluoride used as the fluorinating agent in the present invention, AgF₂ is particularly preferable from the viewpoint of good reactivity.

The organic salt used as a fluorinating agent in the oxidative fluorination reaction is not particularly limited as long as it is an organic salt including a quaternary ammonium cation or a quaternary phosphonium cation. Examples of the organic salt include compounds represented by the following general formulas (s1) to (s7).

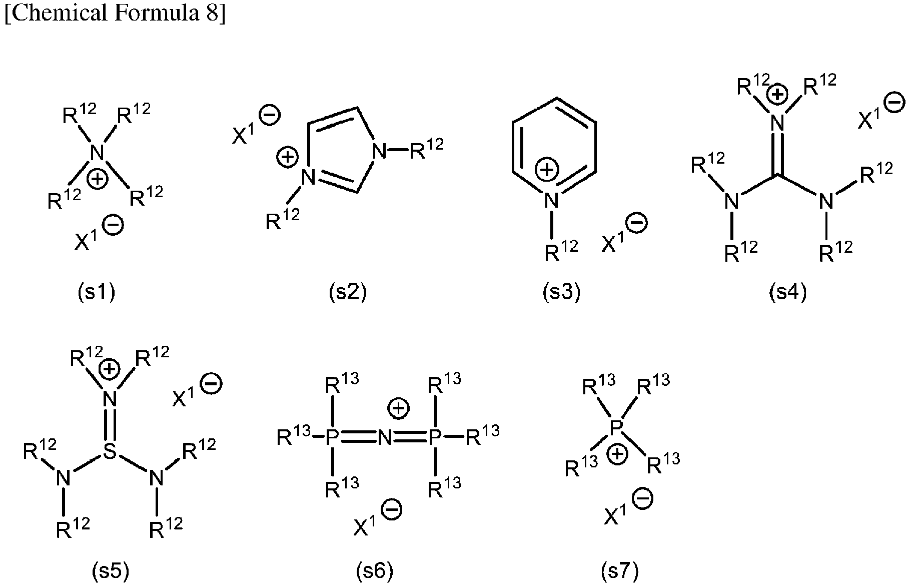

In the general formulas (s1) to (s5), R¹² is a C₁₋₆ alkyl group or an aryl group. The aryl group may be the same group as those mentioned above for A¹. A plurality of R¹² in one molecule may all be the same or different.

In the general formulas (s6) to (s7), R¹³ is a C₁₋₆ alkyl group, an aryl group, a C₁₋₆ alkoxy group or a C₁₋₆ alkylamino group. The aryl group may be the same group as those mentioned above for A¹. The C₁₋₆ alkylamino group is not particularly limited as long as it is a group in which one or two hydrogen atoms of an amino group are substituted with a C₁₋₆ alkyl group. Examples of the C₁₋₆ alkylamino group include a dimethylamino group. A plurality of R¹³ in one molecule may all be the same or different.

In the general formulas (s1) to (s7), X¹ is not particularly limited as long as it is a monovalent anion that forms a salt with a quaternary ammonium cation or a quaternary phosphonium cation. The X¹⁻ includes an iodine ion (I⁻), bromide ion (Br⁻), chloride ion (Cl), fluoride ion (F⁻), hydrogen difluoride ion (HF₂⁻), tribromide ions (Br3-), azide ion (N₃⁻), cyanide ion (CN⁻), cyanate ion (OCN⁻) and the like.

As the organic salt used as the fluorinating agent in the oxidative fluorination reaction, a tetraalkylammonium halide (NR¹¹₄X) is particularly preferable. NR¹¹₄X used in the oxidative fluorination reaction is not particularly limited as long as it is a halide in which four alkyl groups are bonded to a nitrogen atom. As the halide, a chloride or a bromide is preferable, and a chloride is particularly preferable. In addition, the alkyl group bonded to the nitrogen atom may be linear or branched, and the four alkyl groups may all be the same or different from each other. The alkyl group is preferably a C₁₋₆ alkyl group, more preferably a methyl group, an ethyl group or a propyl group. Among them, NR¹¹₄X is preferably N(Et)₄Cl (tetraethylammonium chloride) (CAS No: 56-34-8) or N(Et)₄Br (tetraethylammonium bromide) (CAS No: 71-91-0), more preferably N(Et)₄Cl.

The amount of the organic salt to be added to the reaction system such as NR¹¹₄X may be at least the stoichiometric amount. From the viewpoint of reaction efficiency and cost, the amount of the organic salt such as NR¹¹₄X used in the oxidative fluorination reaction is preferably 1 to 10 equivalents, more preferably 1 to 6 equivalents, with respect to the thioaryl compound (2).

Although the amount of the divalent or higher metal fluoride to be added to the reaction system, such as AgF₂ or the like, is not particularly limited, from the viewpoint of reaction efficiency, it is preferably 5 equivalents or more, more preferably 8 equivalents or more and even more preferably 10 equivalents or more, with respect to the thioaryl compound (2). From the viewpoint of cost, the amount of the divalent or higher valent metal fluoride used in the oxidative fluorination reaction, such as AgF₂ or the like, is preferably 100 equivalents or less, more preferably 50 equivalents or less, even more preferably 30 equivalents or less, and still even more preferably 20 equivalents or less, with respect to the thioaryl compound (2).

The oxidative fluorination reaction can be carried out in a solvent inert to the reaction. Although the inert solvent is not particularly limited, an aprotic polar solvent is preferable. Examples of the aprotic polar solvent include acetonitrile (MeCN), N,N'-dimethylformamide (DMF), N,N-dimethylacetamide, dimethylsulfoxide (DMSO), tetrahydrofuran (THF), dichloromethane (DCM), diethyl ether and the like. The solvent used for the reaction may be a mixed solvent of two or more solvents.

In the oxidative fluorination reaction, a reaction solution is prepared by mixing a thioaryl compound (2), an organic salt such as NR¹¹₄X and a divalent or higher valent metal fluoride such as AgF₂ in a reaction solvent, and reacting at an appropriate temperature and time. The oxidative fluorination reaction proceeds under mild conditions. For example, the reaction temperature is not particularly limited as long as it is a temperature at which the reaction solvent is liquid, and it can be carried out at -40 to 130°C, preferably at 0 to 80°C, and can also be carried out at room temperature (0 to 30°C). For example, the oxidative fluorination reaction can be carried out at room temperature for less than 1 hour, thereby obtaining the desired SF₅-containing aryl compound (1) in an essentially quantitative yield.

By the oxidative fluorination reaction, the divalent or higher valent metal fluoride such as AgF₂ is defluorinated to produce a metal such as Ag. The metal such as Ag or the like produced by the reaction can be recovered and fluorinated to regenerate divalent or higher valent metal fluorides such as AgF₂ or the like. The regenerated divalent or higher valent metal fluoride such as AgF₂ can be used again in the oxidative fluorination reaction. Fluorination of metals such as Ag or the like can be carried out by conventional methods such as heating in fluorine gas.

The oxidative fluorination reaction enables the one-pot synthesis of SF₅-containing aryl compounds (1) in a single step under relatively mild reaction conditions with high yields. In this oxidative fluorination reaction, partially-fluorinated fluorides such as SF₄Cl group-containing aryl compound are not produced in most cases. Therefore, it is an advantage that it is not necessary to isolate an SF₄Cl group-containing aryl compound from the reaction product and purify the desired SF₅-containing aryl compound (1).

### <Thiolation reaction of aryl>

A commercially available compound may be used as the thioaryl compound (2), and a product synthesized by reacting a compound represented by the following general formula (3) with a compound represented by the following general formula (4) may also be used. When the thioaryl compound (2) synthesized by the thiolation reaction of aryl is used for the oxidative fluorination reaction, the thiolation reaction of aryl and the subsequent oxidative fluorination reaction can also be carried out in one pot.

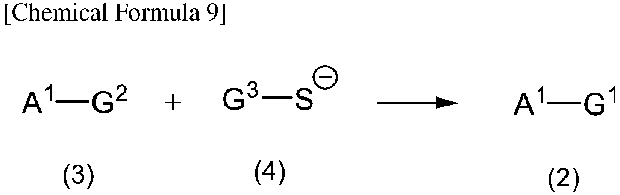

In the general formula (3), A¹ is the same as described above. In addition, G² is a halogen atom, a hydroxy group, or an amino group.

In the general formula (4), G³ is a group obtained by removing a sulfur atom from G2.

The thiolation reaction of an aryl compound can be carried out in a solvent inert to the reaction at a temperature at which the reaction solvent is liquid. As the inert solvent, the same inert solvents as those used in the oxidative fluorination reaction can be used.

### [EXAMPLES]

The present invention will be described below with reference to the Examples, but the present invention is not limited to these Examples.

The NMR equipment used for the analysis of the Examples and the Comparative Examples was JNM-ECZ400S (400 MHz) manufactured by JEOL Ltd. Tetramethylsilane was set at 0 PPM for ¹H NMR, and C₆F₆ was set at -162 PPM for ¹⁹F NMR. In addition, the NMR yield of the obtained SF₅-containing aryl compound was determined in such a manner that after 1,4-bis(trifluoromethyl)benzene (BTB) or 1,4-difluorobenzene (DFB) (0.5 or 1 equivalent with respect to the SF5-containing aryl compound produced) was added to the reaction mixture as an internal standard, the resulting mixture was filtered with a PTFE syringe filter and administered to an NMR tube, followed by determining the NMR yield using the integrated value of the ¹⁹F NMR signals of each of the SF₅ and CF₃ groups.

### [Example 1]

Pentafluorosulfanylation of aryl disulfide was carried out as follows.

First, in an argon-filled glove box, a glass vial was charged with NEt₄Cl (2 eq., 0.2 mmol), aryl disulfide (1 eq., 0.1 mmol), and a magnetic stir bar, followed by addition of MeCN (1mL). The reaction mixture was then stirred until all compounds dissolved (usually 2 minutes). AgF₂ (16 eq, 1.6 mmol) was then added in one portion while the reaction mixture was vigorously stirred at room temperature and the vial was closed with a screw cap. Within about 1 minute of closing the cap, the initially black suspension turned orange with a reddish-purple organic layer. Stirring was continued until the organic layer turned colorless and the oxidative fluorination reaction was complete (usually 2 to 4 hours). The NMR yield of the resulting SF₅-containing aryl compound was determined by adding 1,4-bis(trifluoromethyl)benzene (3 µL, 0.02 mmol) as an internal standard to the reaction mixture.

### <Synthesis of phenylsulfapentafluoride>

The above synthesis reaction was carried out using phenyl disulfide (21.8 mg, 0.1 mmol) as an aryl disulfide to obtain phenyl sulfapentafluoride with an NMR yield of >95%.

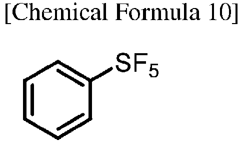

¹H NMR (500 MHz, CD₃CN, 298 K, δ): 8.02 - 7.94 (m, 2H), 7.82 - 7.62 (m, 3H). ¹⁹F {¹H} NMR (471 MHz, CD₃CN, 298 K, δ): 85.38 (p, J = 147.7 Hz, 1F), 62.82 (d, J = 147.7 Hz, 4F).

### <Synthesis of 4-methoxyphenylsulfapentafluoride>

The above synthesis reaction was carried out using 4-methoxyphenyl disulfide (55.7 mg, 0.2 mmol) as an aryl disulfide to obtain 4-methoxyphenylsulfapentafluoride in an NMR yield of >95%.

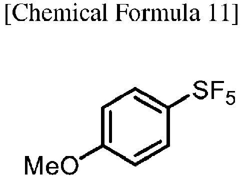

¹H NMR (400 MHz, CD₃CN, 298 K, δ): 7.92 (d, J = 9.3 Hz, 2H), 7.19 (d, J = 9.0 Hz, 2H), 4.01 (s, 3H).
¹⁹F {¹H} NMR (376 MHz, CD₃CN, 298 K, δ): 86.63 (p, J = 148 Hz, 1F), 64.13 (d, J = 148.0 Hz, 4F).

### <Synthesis of 4-nitrophenylsulfapentafluoride>

The above synthesis reaction was carried out using 4-nitrophenyl disulfide (61.7 mg, 0.2 mmol) as an aryl disulfide to obtain 4-nitrophenylsulfapentafluoride in an NMR yield of >95%.

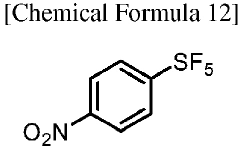

¹H NMR (400 MHz, CD₃CN, 298 K, δ): 8.61 - 8.39 (m, 2H), 8.32 - 8.11 (m, 2H). ¹⁹F {¹H} NMR (376 MHz, CD₃CN, 298 K, δ): 81.61 (p, J = 148.6 Hz, 1F), 62.26 (d, J = 148.6 Hz, 4F).

### <Synthesis of 4-chlorophenylsulfapentafluoride>

The above synthesis reaction was carried out using 4-chlorophenyl disulfide (57.4 mg, 0.2 mmol) as an aryl disulfide to obtain 4-chlorophenylsulfapentafluoride in an NMR yield of >95%.

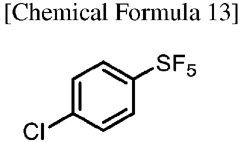

¹H NMR (400 MHz, CD₃CN, 298 K, δ): 7.98 (d, J = 9.0 Hz, 1H), 7.73 (d, J = 8.4 Hz, 1H). ¹⁹F {¹H} NMR (376 MHz, CD₃CN, 298 K, δ): 83.37 (p, J = 149.1 Hz, 1F), 62.39 (d, J = 149.1 Hz, 4F).

### <Synthesis of 2-pyridinylsulfapentafluoride >

The above synthesis reaction was carried out using 2-pyridinyl disulfide (44.1 mg, 0.2 mmol) as an aryl disulfide to obtain 2-pyridinylsulfapentafluoride in an NMR yield of >95%.

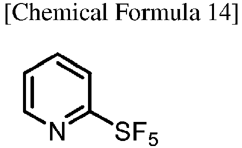

¹H NMR (400 MHz, CD₃CN, 298 K, δ): 8.80 - 8.69 (m, 1H), 8.24 (t, J = 7.9 Hz, 1H), 8.03 (d, J = 8.3 Hz, 1H), 7.86 - 7.76 (m, 1H).
¹⁹F {¹H} NMR (376 MHz, CD₃CN, 298 K, δ): 78.56 (p, J = 147.4 Hz, 1F), 51.16 (d, J = 147.4 Hz).

### <Synthesis of 4-methylphenylsulfapentafluoride>

The above synthesis reaction was carried out using 4-methylphenyl disulfide (24.4 mg, 0.1 mmol) as an aryl disulfide, and allowed to react for 4 hours to obtain 4-methylphenylsulfapentafluoride with an NMR yield of 95%.

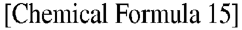

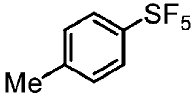

¹H NMR (400 MHz, CD₃CN) δ7.73 - 7.64 (m, 2H), 7.34 (d, J = 8.1 Hz, 2H), 2.38 (s, 3H). ¹³C NMR (101 MHz, CD₃CN) δ 152.1 (d, J = 15.3 Hz), 144.3 - 143.9 (m), 130.6 (s), 126.7 (p, ³J_{CF} = 4.7 Hz), 21.2 (s).
¹⁹F NMR (376 MHz, CD₃CN) δ 85.4 (p, ²JFF,ax = 147.8 Hz, 1F, SF), 62.6 (d, ²J_{FF,eq} = 147.8 Hz, 4F, SF₄).

### <Synthesis of N,N-bis (tert-butoxycarbonyl)-4-aminophenylsulfapentafluoride>

The above synthesis reaction was carried out using N,N-bis (tert-butoxycarbonyl)-4-aminophenyl disulfide (65.0 mg, 0.1 mmol) as an aryl disulfide, and allowed to react for 4 hours to obtain N,N-bis (tert-butoxycarbonyl)-4-aminophenylsulfapentafluoride with an NMR yield of 88%.

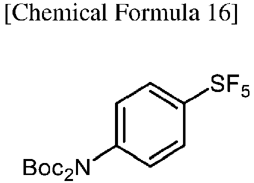

¹H NMR (400 MHz, CD₃CN) 7.86 - 7.81 (m, 2H), 7.37 (d, J = 9.0 Hz, 2H), 1.40 (s, 18H). ¹³C NMR (101 MHz, CD₃CN) δ 152.9 - 152.4 (m), 152.1 (s), 144.0 - 143.7 (m), 129.6 (s), 127.5 (p, ³J_{CF} = 4.7 Hz), 84.1 (s), 28.0 (s).
¹⁹F NMR (376 MHz, CD₃CN) δ 84.1 (p, ²JFF,ax = 148.2 Hz, 1F, SF), 62.6 (d, ²J_{FF,eq} = 147.9 Hz, 4F, SF₄).

### [Example 2]

Pentafluorosulfanylation of free thiophenol was carried out as follows.

First, in an argon-filled glove box, a glass vial was charged with NEt₄Cl (2 to 3 eq., 0.4 to 0.6 mmol), thiophenol (1 eq., 0.2 mmol), and a magnetic stir bar, followed by addition of MeCN (1mL). The reaction mixture was then stirred until all compounds dissolved (usually 2 minutes). AgF₂ (10 eq, 2 mmol) was then added in one portion while the reaction mixture was vigorously stirred at room temperature and the vial was closed with a screw cap. Within about 1 minute of closing the cap, the initially black suspension turned orange with a reddish-purple organic layer. Stirring was continued until the organic layer turned colorless and the oxidative fluorination reaction was complete (usually 4 to 36 hours). The NMR yield of the resulting SF₅-containing aryl compound was determined by adding 1,4-bis(trifluoromethyl)benzene (3 µL, 0.02 mmol) as an internal standard to the reaction mixture. Table 1 shows the results.

### [Example 3]

Pentafluorosulfanylation of (hetero)aryl thiocyanate can be carried out, for example, by the following method.

First, in an argon-filled glove box, a PFA vial was charged with NEt₄Cl (66.3 mg, 0.4 mmol, 2 eq.), the corresponding (hetero)arylthiocyanate (0.2 mmol), MeCN (1 mL) and a magnetic stirrer bar. AgF₂ (292 mg, 2.0 mmol, 10 eq.) was then added to the stirring reaction in the vial at room temperature and the vial was closed with a screw cap. Within about 1 minute of closing the cap, the reaction solution, which was initially a black suspension, changed to form an orange precipitate with a reddish-purple organic layer. The reaction solution was stirred for 24 hours to react.

Pentafluorosulfanylation of phenyl thiocyanate (phenylthiocyanate), among (hetero)aryl thiocyanates, was carried out as follows.

First, in an argon-filled glove box, a glass vial was charged with NEt₄Cl (2 to 3 eq., 0.4 to 0.6 mmol), phenyl thiocyanate (1 eq., 0.2 mmol), and a magnetic stir bar, followed by addition of MeCN (1mL). The reaction mixture was then stirred until all compounds dissolved (usually 2 minutes). AgF₂ (12 eq, 2.4 mmol) was then added in one portion while the reaction mixture was vigorously stirred at room temperature and the vial was closed with a screw cap. Within about 1 minute of closing the cap, the initially black suspension turned orange with a reddish-purple organic layer. Stirring was continued until the organic layer turned colorless and the oxidative fluorination reaction was complete (usually 30 minutes to 1 hour). The NMR yield of the resulting SF₅-containing aryl compound was determined by adding 1,4-bis(trifluoromethyl)benzene (3 µL, 0.02 mmol) as an internal standard to the reaction mixture. Table 1 shows the results.

### [Example 4]

Pentafluorosulfanylation of benzoyl-protected thiophenols (phenylthiol benzoates) was carried out as follows.

First, in an argon-filled glove box, a glass vial was charged with NEt₄Cl (1 to 2 eq., 0.1 to 0.2 mmol), benzoylthiophenol (1 eq., 0.2 mmol), and a magnetic stir bar, followed by addition of MeCN (1mL). The reaction mixture was then stirred until all compounds dissolved (usually 2 minutes). AgF₂ (10 eq, 2 mmol) was then added in one portion while the reaction mixture was vigorously stirred at room temperature and the vial was closed with a screw cap. Within about 1 minute of closing the cap, the initially black suspension turned orange with a reddish-purple organic layer. Stirring was continued until the organic layer turned colorless and the oxidative fluorination reaction was complete (usually 2 to 24 hours). The NMR yield of the resulting SF₅-containing aryl compound was determined by adding 1,4-bis(trifluoromethyl)benzene (3 µL, 0.02 mmol) as an internal standard to the reaction mixture. Table 1 shows the results.

**[Table 1]**

| Ex. | Reactant | NEt₄Cl | Reaction Condition | Yield of SF₅-containing aryl compound |
|---|---|---|---|---|
| 2 | Thiophenol | 2 eq. | Room temperature, 48 hours | >95% |
| | | 3 eq. | | >95% |
| 3 | Phenyl thiocyanate | 2 eq. | Room temperature, 24 hours | 68% |
| | | 3 eq. | | 55% |
| 4 | S-phenylthiobenzoate | 1 eq. | Room temperature, 24 hours | 95% |
| | | 2 eq. | | 84% |
| | S-(4-methoxyphenyl) thiobenzoate | 1 eq. | Room temperature, 24 hours | >95% |
| | | 2 eq. | | >95% |

### [Example 5]

Synthesis of arylpentafluorosulfanyl compound from a benzenediazonium salt was carried out using copper-catalyzed Sandmeyer coupling/oxidative fluorination.

### (1) Synthesis of tetraethylammonium thiobenzoate

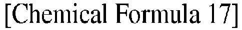

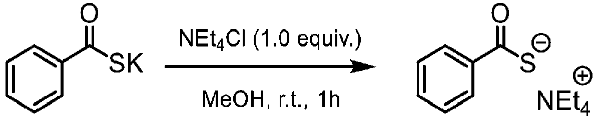

In a reaction vessel under nitrogen atmosphere, potassium thiobenzoate (891 mg, 5.05 mmol) and tetraethylammonium chloride (818 mg, 4.95 mmol) were dissolved in dry MeOH (5 mL) and stirred at room temperature for 1 hour. After filtering off the KCl precipitate with a syringe filter, the solvent was further evaporated in vacuo. The reaction vessel was then transferred to a glove box and the solid in the reaction vessel was dissolved in MeCN (10 mL). The resulting solution was filtered with a syringe filter followed by concentration in vacuo to obtain a yellow solid as a product (1.28 g, yield of 96%).

¹H NMR (500 MHz, d₃-MeCN) δ 8.18 - 8.07 (m, 2H), 7.31 - 7.19 (m, 3H), 3.16 (q, *J* = 7.3 Hz, 8H), 1.19 (tt, *J* = 7.3 Hz, 1.8 Hz, 12H).

### (2) Synthesis of (phen) CuSCOPh

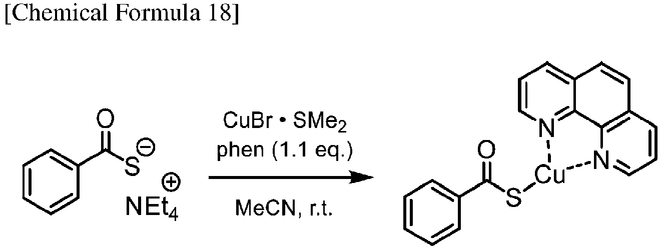

Copper (I) bromide dimethylsulfide complex (205 mg, 1 mmol) and tetraethylammonium thiobenzoate (295 mg, 1.1 mmol) were dissolved in MeCN (5 mL). The resulting yellow solution was filtered with a syringe filter and diluted with MeCN (5 mL). Then 1,10-phenanthroline (198 mg, 1.1 mmol) was added portionwise to the solution as a solid to obtain a red precipitate. The resulting red precipitate was filtered off, washed with MeCN and Et₂O, followed by drying in vacuo to isolate a red powder (360 mg, yield of 95%) as a product.

### (3) Copper-catalyzed Sandmeyer coupling/oxidative fluorination reaction

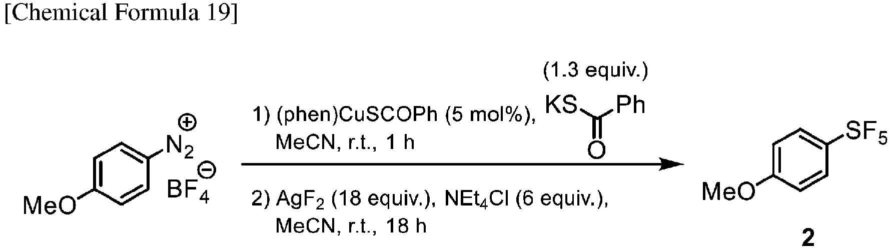

In an argon-filled drybox, a glass vial was charged with (phen)CuSCOPh (3.8 mg, 0.01 mmol), potassium thiobenzoate (45.8 mg, 0.26 mmol), and a magnetic stir bar, followed by addition of MeCN (0.5 mL). The mixture was then stirred at room temperature. After stirring for 2 minutes, a red solution containing undissolved potassium thiobenzoate was obtained. Then, 4-methoxybenzenediazonium tetrafluoroborate (44.4 mg, 0.2 mmol) dissolved in MeCN (0.5 mL) was added dropwise to the red solution using a syringe at room temperature. Generation of nitrogen gas was visually observed during the addition. The syringe was washed using 0.25 mL of MeCN and the washing liquid was also added to the reaction mixture. The reaction mixture was stirred at room temperature for 1 hour, 1,4-bis(trifluoromethyl)benzene (31 µL, 0.2 mmol) was added as an internal standard, then filtered with a syringe filter. The syringe was washed using 0.25 mL of MeCN. Tetraethylammonium chloride (106 mg, 1.2 mmol) was added to the reaction mixture and dissolved (stirring for about 2 minutes). Subsequently, silver (11) fluoride (525 mg, 3.6 mmol) was added to the reaction mixture and stirred overnight at room temperature. The NMR yield (78%) of pentafluorosulfanyl arene ("2" in the formula) was determined by ¹⁹F NMR spectroscopy by comparison with the internal standard.

¹H NMR (400 MHz, d₃-MeCN) δ 7.92 (d, *J* = 9.3 Hz, 2H), 7.19 (d, *J* = 9.0 Hz, 2H), 4.01 (s, 3H).
¹⁹F {1H} NMR (376 MHz, d₃-MeCN) δ 86.63 (p, *J* = 148 Hz, 1F), 64.13 (d, *J* = 148.0 Hz, 4F).

### [Example 6]

Pentafluorosulfanylation of (hetero)arylthiol can be carried out, for example, by the following method.

First, in an argon-filled glovebox, a PFA vial was charged with NEt₄Cl (66.3 mg, 0.4 mmol, 2 eq), the corresponding (hetero) arylthiol (0.2 mmol), MeCN (1 mL) and a magnetic stirrer bar. AgF₂ (292 mg, 2.0 mmol, 10 eq) was then added to the stirring reaction solution in the vial at room temperature and the vial was closed with a screw cap. Within about 1 minute of closing the cap, the reaction solution, which was initially a black suspension, changed to form an orange precipitate with a reddish-purple organic layer. The reaction solution was stirred for 4 to 36 hours to react.

### <Synthesis of 4-methoxyphenylsulfapentafluoride>

The above synthesis reaction was carried out using 4-methoxythiophenol (25 µL, 0.2 mmol) as the (hetero) arylthiol, and allowed to react for 36 hours to obtain 4-methoxyphenylsulfapentafluoride with an NMR yield of 42%.

### <Synthesis of 4-nitrophenylsulfapentafluoride>

The above synthesis reaction was carried out using 4-nitrophenylthiol (30.6 mg, 0.1 mmol) as the (hetero) arylthiol, and allowed to react for 4 hours to obtain 4-nitrophenylsulfapentafluoride with an NMR yield of 99%.

### <Synthesis of 4-bromophenylsulfapentafluoride>

The above synthesis reaction was carried out using 4-bromophenylthiol (37.8 mg, 0.2 mmol) as the (hetero) arylthiol, and allowed to react for 36 hours to obtain 4-bromophenylsulfapentafluoride with an NMR yield of 98%.

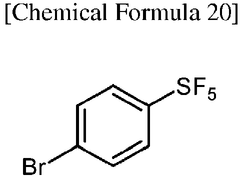

¹H NMR (500 MHz, CD₃CN) δ 7.78 -7.65 (m, 4H).
¹³C NMR (126 MHz, CD₃CN) δ 154.0 - 152.6 (m), 133.4 (s), 128.8 (p, ³J_{CF} = 4.7 Hz), 127.2 - 127.1 (m).
¹⁹F NMR (471 MHz, CD₃CN) δ 83.5 (p, ²J_{FF,ax} = 147.9 Hz, 1F, SF), 62.5 (d, ²J_{FF,eq} = 147.9 Hz, 4F, SF₄).

### <Synthesis of 4-trifluoromethylphenylsulfapentafluoride>

The above synthesis reaction was carried out using 4-trifluoromethylphenylthiol (24 µL, 0.2 mmol) as the (hetero) arylthiol, and allowed to react for 36 hours to obtain 4-trifluoromethylphenylsulfapentafluoride with an NMR yield of 99%. 1,4-Difluorobenzene (DFB) (10 µL, 0.1 mmol) was used as an internal standard.

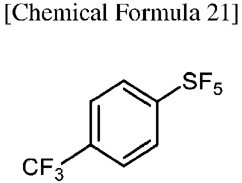

¹H NMR (500 MHz, CD₃CN) δ 8.03 (d, J = 8.6 Hz, 2H), 7.89 (d, J = 8.4 Hz, 2H).
¹³C NMR (126 MHz, CD₃CN) δ 156.9 (t, ²J_{CF} = 17.9 Hz), 134.2 (q, ²J_{CF} = 33.8 Hz), 128.1 (p, ³J_{CF} = 4.8 Hz), 127.6 (q, ³J_{CF} = 3.8 Hz), 124.4 (q, ¹J_{CF} = 272.0 Hz).
¹⁹F NMR (471 MHz, CD₃CN) δ 82.2 (p, ²J_{FF,ax} = 148.3 Hz, 1F, SF), 61.7 (d, ²J_{FF,eq} = 148.0 Hz, 4F, SF₄), -63.7 (s, 3F, CF₃).

### <Synthesis of 3,5-bis(trifluoromethyl)phenylsulfapentafluoride>

The above synthesis reaction was carried out using 3,5-bis(trifluoromethyl) phenylthiol (34 µL, 0.2 mmol) as the (hetero) arylthiol, and allowed to react for 36 hours to obtain 3,5-bis(trifluoromethyl)phenylsulfapentafluoride with an NMR yield of 99%. 1,4-Difluorobenzene (10 µL, 0.1 mmol) was used as an internal standard.

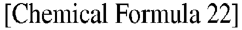

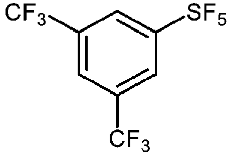

¹H NMR (500 MHz, CD₃CN) δ 8.40 (s, 2H), 8.28 (s, 1H).
¹³C NMR (126 MHz, CD₃CN) δ 155.1 - 154.5 (m), 133.3 (q, ²J_{CF} = 34.6 Hz), 128.2 (q, ³J_{CF} = 4.1 Hz), 127.7 (p, ³J_{CF} = 4.0 Hz), 123.66 (q, ¹J_{CF} = 272.7 Hz).
¹⁹F NMR (471 MHz, CD₃CN) δ 80.0 (p, ²J_{FF,ax} = 149.9 Hz, 1F, SF), 62.3 (d, ²J_{FF,eq} = 149.9 Hz, 4F, SF₄), -63.4 (s, 6F, CF₃).

### <Synthesis of 4-(pentafluorosulfanyl)benzoyl fluoride>

The above synthesis reaction was carried out using 4-mercaptobenzoyl chloride (32.1 mg, 0.2 mmol) as the (hetero) arylthiol, and allowed to react for 36 hours to obtain 4-(pentafluorosulphanyl)benzoyl fluoride with an NMR yield of 91%.

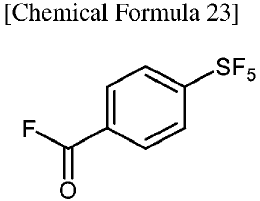

¹H NMR (500 MHz, CD₃CN) δ 8.24 - 8.17 (m, 2H), 8.07 - 8.01 (m, 2H).
¹⁹F NMR (471 MHz, CD₃CN) δ 81.6 (p, ²J_{FF,ax} = 148.3 Hz, 1F, SF), 61.5 (d, ²J_{FF,eq} = 148.5 Hz, 4F, SF₄), 19.1 (s, 1F, COF).

### <Synthesis of 4,4'-bis(pentafluorosulfanyl)-1,1'-biphenyl>

A synthesis reaction was carried out using 4,4'-biphenyldithiol (43.7 mg, 0.2 mmol) as the (hetero)arylthiol, NEt₄Cl (132 mg, 0.8 mmol, 4 eq.) and AgF₂ (584 mg, 4 mmol, 20 eq.), and allowed to react for 36 hours. The reaction mixture was then filtered and the residue was washed with MeCN. The filtrate containing the washing liquid was concentrated in vacuo and the crude product was purified by column chromatography (hexane) to obtain 4,4'-bis (pentafluorosulfanyl)-1,1'-biphenyl as a colorless solid (46 mg, yield of 57%).

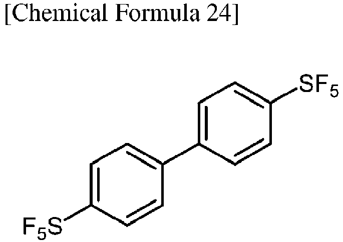

¹H NMR (400 MHz, benzene-d₆) δ 7.50 - 7.33 (m, 4H), 6.85 (d, J = 8.8 Hz, 4H).
¹³C NMR (101 MHz, benzene-d₆) δ 153.8 (p, ²J_{CF} = 17.5 Hz), 142.2 (s), 127.6 (s), 126.7 (p, ³J_{CF} = 4.6 Hz).
¹⁹F NMR (376 MHz, benzene-d₆) δ 84.9 (p, ²J_{FF,ax} = 152.4 Hz, 1F, SF), 63.3 (d, ²J_{FF,eq} = 152.4 Hz, 4F, SF₄).

### [Example 7]

Pentafluorosulfanylation of (hetero) arylthiol benzoate (benzoyl-protected (hetero) arylthiol) can be carried out, for example, by the following method.

First, in an argon-filled glovebox, a PFA vial was charged with NEt₄Cl (33.1 mg, 0.2 mmol, 1 eq.), the corresponding (hetero) arylthiol benzoate (0.2 mmol), MeCN (1 mL) and a magnetic stirrer bar. AgF₂ (292 mg, 2.0 mmol, 10 eq.) was then added to the stirring reaction in the vial at room temperature and the vial was closed with a screw cap. Within about 1 minute of closing the cap, the reaction solution, which was initially a black suspension, changed to form an orange precipitate with a reddish-purple organic layer. The reaction solution was stirred for 2 to 24 hours to react.

### <Synthesis of 4-methoxyphenylsulfapentafluoride>

The above synthesis reaction was carried out using 4-methoxyphenylthiobenzoate (49.2 mg, 0.2 mmol) as the (hetero) aryl thiol benzoate, and allowed to react for 2 hours to obtain 4-methoxyphenylsulfapentafluoride with an NMR yield of 76%.

### [Example 8]

Pentafluorosulfanylation of (hetero) arylthiol tritylate can be carried out, for example, by the following method.

First, in an argon-filled glovebox, a PFA vial was charged with the corresponding (hetero) arylthiol tritylate (0.2 mmol), MeCN (1 mL), and a magnetic stirrer bar. AgF₂ (175 mg, 1.2 mmol, 12 eq) was then added to the stirring reaction in the vial at room temperature and stirred for an additional hour. NEt₄Cl (16.7 mg, 0.1 mmol, 1 eq) was then added to the vial and the vial was closed with a screw cap. The reaction solution was stirred for 2 to 24 hours to react.

### <Synthesis of 4-methoxyphenylsulfapentafluoride>

The above synthesis reaction was carried out using (4-methoxyphenyl) (trityl) sulfane (38.4 mg, 0.1 mmol) as the (hetero) arylthiol tritylate, and allowed to react for 2 hours to obtain 4-methoxyphenylsulfapentafluoride with an NMR yield of 88%.

### [Industrial applicability]

The present invention provides a production method that allows the synthesis of an SF₅-containing aryl compound in a single step under relatively mild conditions. In addition, in the oxidative fluorination reaction according to the present invention, an SF₅ group can be introduced into various aryl compounds. Therefore, the present invention is useful for introducing an SF₅ group into active ingredients of pharmaceuticals and agricultural chemicals, organic materials and the like.

## Claims

1. A method for producing a pentafluorosulfanyl group-containing aryl compound, comprising:
synthesizing a pentafluorosulfanyl group-containing aryl compound represented by the following general formula (1) from a thioaryl compound represented by the following general formula (2) by an oxidative fluorination reaction using a divalent or higher valent metal fluoride and an organic salt including a quaternary ammonium cation or a quaternary phosphonium cation,
A¹-G¹ (2)
[in the formula, A¹ is an optionally substituted aryl group or an optionally substituted heteroaryl group; -G¹ is -SH, -SCN, -SF₃, -S-S-R¹ (R¹ is an optionally substituted aryl group or an optionally substituted heteroaryl group), -S-CO-R² (R² is an optionally substituted aryl group, an optionally substituted heteroaryl group, an optionally substituted alkyl group having 1 to 6 carbon atoms or an optionally substituted alkenyl group having 2 to 6 carbon atoms), -S-R³ (R³ is an optionally substituted alkyl group having 1 to 6 carbon atoms or an optionally substituted alkenyl group having 2 to 6 carbon atoms), -SF₂-R⁴ (R⁴ is an optionally substituted alkyl group having 1 to 6 carbon atoms or an optionally substituted alkenyl group having 2 to 6 carbon atoms), -S-Si-(R⁵)₃ (R⁵ is an optionally substituted aryl group, an optionally substituted heteroaryl group, an optionally substituted alkyl group having 1 to 6 carbon atoms, or an optionally substituted alkenyl group having 2 to 6 carbon atoms, and a plurality of R⁵ may be the same or different), -S-PO-(R⁶)₂ (R⁶ is an optionally substituted aryloxy group, an optionally substituted heteroaryloxy group, an optionally substituted alkyloxy group having 1 to 6 carbon atoms, an optionally substituted alkenyloxy group having 2 to 6 carbon atoms, an optionally substituted aryl group, an optionally substituted hetero aryl group, an optionally substituted alkyl group having 1 to 6 carbon atoms, or an optionally substituted alkenyl group having 2 to 6 carbon atoms, and a plurality of R⁶ may be the same or different), an optionally substituted (N-phthalimidyl) thio group, or an optionally substituted thianthrenium group],
A¹-SF₅ (1)
[in the formula, A¹ is the same as described above].

2. The method for producing a pentafluorosulfanyl group-containing aryl compound according to Claim 1, wherein A¹ is
an aryl group optionally substituted with one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a cyano group, a fluoroformyl group, an amino group and a nitro group; or
a heteroaryl group optionally substituted with one or more substituents selected from the group consisting of a halogen atom, an alkyl group, a fluorinated alkyl group, an alkenyl group, an aryl group, a heteroaryl group, an alkoxy group, a hydroxy group, a carboxy group, an acyl group, a fluoroformyl group, a cyano group, an amino group and a nitro group.

3. The method for producing a pentafluorosulfanyl group-containing aryl compound according to Claim 1 or 2, wherein the oxidative fluorination reaction is carried out at -40 to 130°C.

4. The method for producing a pentafluorosulfanyl group-containing aryl compound according to any one of Claim 1 to 3, wherein a metal produced after the oxidative fluorination reaction is recovered.

5. The method for producing a pentafluorosulfanyl group-containing aryl compound according to Claim 4, wherein
the recovered metal is fluorinated to regenerate a divalent or higher valent metal fluoride, and
the obtained divalent or higher valent metal fluoride is used again in the oxidative fluorination reaction.

6. The method for producing a pentafluorosulfanyl group-containing aryl compound according to any one of Claim 1 to 5, wherein the divalent or higher valent metal fluoride is silver (II) fluoride.

7. The method for producing a pentafluorosulfanyl group-containing aryl compound according to any one of Claim 1 to 6, wherein the organic salt is a tetraalkylammonium halide.
